# EUROPEAN PATENT APPLICATION

(11) **EP 4 696 234 A1**
(43) Date of publication of application: **18.02.2026**
(21) Application number: 24814336.4
(22) Date of filing: 24.05.2024
(51) Int. Cl.: A61B 5/1455

(54) **OXIMETER REGULATION METHOD AND APPARATUS**

(30) Priority: 26.05.2023 CN 202310607892
(71) Applicant: Beijing Choice Electronic Technology Co., Ltd., Beijing 100041 (CN)
(72) Inventor: QIAN, Xiaolun, Beijing 100041 (CN); MA, Chuanlong, Beijing 100041 (CN); ZHENG, Lijin, Beijing 100041 (CN)
(74) Representative: Arnold & Siedsma
(86) International application number: PCT/CN2024/095189
(87) International publication number: WO 2024/245138

(57) **Abstract**

Provided in the embodiments of the present disclosure are an oximeter regulation method and apparatus. The method comprises: acquiring light intensity data corresponding to a target finger, so as to form a light intensity data set, the light intensity data being light intensity data of light transmitting through the target finger measured by a light receiving component of an oximeter; According to state information correcting to the light intensity data set, calculating parameter information correcting to the light intensity data set; and according to the parameter information, regulating the light intensity of a light-emitting component of the oximeter. The present disclosure solves the problems such as narrow application ranges and inaccurate measurement results of oximeters in the prior art due to the influences of different human finger thicknesses, skin colors and environmental factors.

## Description

The present application claims priority of a Chinese patent application No. 202310607892.0 filed to the CNIPA on May 26, 2023, and entitled "OXMETER REGULATION METHOD AND APPARATUS", the entire disclosure of which is incorporated herein by reference.

### Technical Field

The present application relates to a method for adjusting an oximeter and an apparatus therefor, and more particularly, to a method for adjusting an oximeter and an apparatus therefor.

### Background

As a portable medical device, users can use an oximeter to detect their health status, including heart rate and blood oxygen, anytime and anywhere. There are two measurement modes for the oximeter: reflective mode and transmissive mode.

The measurement principle of the transmissive oximeter is as follows: a light-emitting LED is on one side of a finger, and a photodiode is on the other side (which is configured to detect light intensity and convert light into a current, Wherein the stronger the light, the greater the current) of the finger. Human's fingers (including skin, bones, and blood) can absorb light, wherein blood absorbs more light than other tissues. During the flow of blood, due to periodic beating of the heart, the blood presents pulse in a pulsatile form, which causes the absorbed light to be different, accordingly there is varied photocurrent on the photodiode. If the sampling frequency is high enough, a complete and continuous pulse waveform will be presented. The heart rate is calculated by counting the quantity of continuous pulse waveforms, so that the quantity of waveforms per minute, i.e., the quantity of heartbeats will be obtained. Oxygen inhaled through breathing combines with hemoglobin in the blood to form oxygenated hemoglobin. Hemoglobin has a higher absorption coefficient for red light, and oxygenated hemoglobin has a higher absorption coefficient for infrared light. Blood oxygen result can be calculated by respectively detecting oxygenated hemoglobin and hemoglobin with red light and infrared light.

At present, main oximeters have some problems, such as a narrow applicable range and inaccurate detection results, due to influences of human finger thickness, skin color and environmental factors during a process of using the oximeters.

### Summary

Embodiments described herein provide a method for adjusting an oximeter, and an apparatus therefor, which solve problems in the existing art.

In a first aspect, according to the present disclosure, there is provided a method for adjusting an oximeter, including:
acquiring light intensity data corresponding to a target finger to form a light intensity data set, wherein the light intensity data is light intensity data transmitted through the target finger and detected by a light receiving component of the oximeter;
calculating parameter information corresponding to the light intensity data set according to state information corresponding to the light intensity data set; and
adjusting a luminous intensity of a light-emitting component of the oximeter according to the parameter information.

**In** some embodiments of the present disclosure, the state information includes a quantity of light intensity data included in the light intensity data set and/or a duration for which the light intensity data set is collected, wherein a type of the parameter information includes a perfusion index and/or an average light intensity.

In some embodiments of the present disclosure, acquiring the light intensity data corresponding to the target finger to form the light intensity data set includes:
collecting the light intensity data corresponding to the target finger, and sequentially forming the light intensity data set of which the state information is a target preset threshold;
determining the parameter information corresponding to the light intensity data set according to the state information corresponding to the light intensity data set includes:
   configuring a pre-associated adjustment parameter type according to the target preset threshold, and calculating parameter information corresponding to the adjustment parameter type according to the acquired light intensity data set.
   In some embodiments of the present disclosure, the target preset threshold includes, at least, a first target preset threshold and a second target preset threshold;
   sequentially forming the light intensity data set of which the state information is the target preset threshold includes:
      sequentially forming a first light intensity data set of which state information is the first target preset threshold, and a second light intensity data set of which state information is the second target preset threshold;
      configuring the pre-associated adjustment parameter type according to the target preset threshold, and calculating the parameter information corresponding to the adjustment parameter type according to the acquired light intensity data set includes:
         determining a perfusion index as the adjustment parameter type according to the first light intensity data set, and calculating a corresponding first perfusion index according to the first light intensity data set;
         determining an average light intensity as the adjustment parameter type according to the second light intensity data set, and calculating a corresponding first average light intensity according to the second light intensity data set.

In some embodiments of the present disclosure, calculating the corresponding first perfusion index according to the first light intensity data set includes:
calculating an average value of the first light intensity data set;
calculating a difference between a maximum light intensity value and a minimum light intensity value corresponding to light intensity data included in the first light intensity data set; and
determining the first perfusion index by the average value and the difference between the maximum light intensity value and the minimum light intensity value.

In some embodiments of the present disclosure, calculating the parameter information corresponding to the light intensity data set according to the state information corresponding to the light intensity data set includes:
when the state information of the acquired light intensity data set meets N times a target preset threshold, configuring a pre-associated adjustment parameter type according to the target preset threshold, and calculating parameter information corresponding to the adjustment parameter type according to a latest acquired light intensity data subset, wherein the light intensity data set includes N light intensity data subsets, state information of each light intensity data subset is the target preset threshold, and N is an integer greater than or equal to 1.

In some embodiments of the present disclosure, the target preset threshold includes, at least, a first target preset threshold and a second target preset threshold;
when the state information of the acquired light intensity data set meets N times the target preset threshold, configuring the pre-associated adjustment parameter type according to the target preset threshold, and calculating the parameter information corresponding to the adjustment parameter type according to the latest acquired light intensity data subset, includes:
when the state information of the acquired light intensity data set meets A times the first target preset threshold, determining a perfusion index as the adjustment parameter type according to the first target preset threshold, and calculating a corresponding second perfusion index according to the latest acquired light intensity data subset;
when the state information of the acquired light intensity data set meets B times the second target preset threshold, determining an average light intensity as the adjustment parameter type according to the second target preset threshold, and calculating a corresponding second average light intensity according to the latest acquired light intensity data subset.

In some embodiments of the present disclosure, the state parameter is a duration for which the light intensity data set is collected, the first target preset threshold is a duration of one cycle of pulse waves, and the second target preset threshold is less than the duration of one cycle of pulse waves;
and/or,
the state parameter is a quantity of collected light intensity data sets, and the second target preset threshold is less than the first target preset threshold.

In some embodiments of the present disclosure, adjusting the luminous intensity of the light-emitting component of the oximeter according to the parameter information includes:
adjusting a current gear of the oximeter according to a relationship between the parameter information and preset parameter information.

In some embodiments of the present disclosure, the current gear of the oximeter includes a plurality of gears, wherein a current value at a first gear is a current value required for a maximum light intensity that a photodiode in the oximeter is capable of receiving when there is no finger in the oximeter, the current value at the first gear is a minimum current value; a current value at a highest gear is a current value required for a maximum light intensity that the photodiode is capable of receiving when a preset dark finger is put into the oximeter, the current value at the highest gear is a maximum current value; current values at other gears are obtained by equally dividing a range between the maximum current value and the minimum current value.

In some embodiments of the present disclosure, adjusting the current gear of the oximeter includes increasing the current gear of the oximeter and decreasing the current gear of the oximeter, and increasing or decreasing the current gear of the oximeter is performed each time by one current gear.

In some embodiments of the present disclosure, adjusting the luminous intensity of the light-emitting component of the oximeter according to the parameter information includes:
adjusting a current gear of the oximeter according to a relationship between the perfusion index and a preset perfusion index; and/or,
adjusting the current gear of the oximeter according to a relationship between the average light intensity and a preset average light intensity.

In some embodiments of the present disclosure, the preset perfusion index includes a first preset perfusion index and a second preset perfusion index;
adjusting the current gear of the oximeter according to the relationship between the perfusion index and the preset perfusion index includes:
increasing the current gear of the oximeter when the perfusion index is less than the first preset perfusion index; and
decreasing the current gear of the oximeter when the perfusion index is greater than the second preset perfusion index.

In some embodiments of the present disclosure, the preset average light intensity includes a first preset average light intensity and a second preset average light intensity; adjusting the current gear of the oximeter according to the relationship between the average light intensity and the preset average light intensity includes:
increasing the current gear of the oximeter when the average light intensity is less than the first preset average light intensity; and
decreasing the current gear of the oximeter when the average light intensity is greater than the second preset average light intensity.

In some embodiments of the present disclosure, the method further includes:
acquiring a preset quantity of light intensity data sets in a sliding window way;
calculating average values of the light intensity data sets; and
determining that the finger falls off and outputting alarm information when an average value is greater than a first preset value and the current gear is a lowest gear.

In a second aspect, according to the present disclosure, there is provided an oximeter adjustment apparatus, including:
a light-emitting component, a light receiving component, and a master control module that is connected to the light-emitting component and the light receiving module;
the master control module is configured to acquire light intensity data received by the light receiving module, to form a light intensity data set, calculate parameter information corresponding to the light intensity data set according to state information corresponding to the light intensity data set, and adjust a luminous intensity of the light-emitting component according to the parameter information.

In a third aspect, according to the present disclosure, there is provided an electronic apparatus including a processor, and a memory having stored therein a computer program that, when executed by the processor, implements the method as described above.

In a fourth aspect, according to the present disclosure, there is provided a computer readable storage medium having stored thereon a computer program that, when executed, implements the method as described above.

According to the method for adjusting an oximeter and the oximeter adjustment apparatus according to the embodiments of the present disclosure, light intensity data corresponding to a target finger is acquired to form a light intensity data set, wherein the light intensity data is light intensity data transmitted through the target finger and detected by a light receiving component of the oximeter; parameter information corresponding to the light intensity data set is calculated according to state information corresponding to the light intensity data set; and a luminous intensity of the light-emitting component of the oximeter is adjusted according to the parameter information, wherein the parameter information corresponding to the calculated light intensity data set includes an average light intensity and a perfusion index. Adjusting the current gear of the oximeter according to the average light intensity can eliminate the influences of bright fingers, dark fingers and ambient light on the measurement accuracy of the oximeter. The current gear of the oximeter is adjusted based on the perfusion index, which eliminates the impact of the human body's intrinsic environment on the measurement accuracy of the oximeter, thereby not only eliminating the influences of both finger thickness and skin color, but also eliminating the influences of environmental factors, on the measurement accuracy of the oximeter. In the present invention, different adjustment parameter types are determined according to the state information of the light intensity data set, which enhances the adjustment efficiency for the light intensity, so that the oximeter can stably output the value in a short time, and the oximeter can output the value faster.

The above description is merely a summary of the technical solutions of the embodiments of the present application. In order to more clearly understand the technical means of the embodiments of the present application, the embodiments of the present application can be implemented according to the contents of the specification. In order to make the above and other objects, features, and advantages of the embodiments of the present application more clearly understood, the specific implementations of the present application are referred to below.

### Brief Description of Drawings

In order to explain technical solutions of embodiments of the present disclosure more clearly, the accompanying drawings of the embodiments will be described briefly below. It should be noted that the accompanying drawings in the following description only relate to some embodiments of the present disclosure, but do not limit the present disclosure.
FIG. 1 illustrates schematically a flowchart of a method for adjusting an oximeter according to an embodiment of the present disclosure.
FIG. 2 illustrates schematically a flowchart of another method for adjusting an oximeter according to an embodiment of the present disclosure.
FIG. 3 is a schematic diagram of a structure of an oximeter adjustment apparatus according to an embodiment of the present disclosure.
FIG. 4 is a schematic diagram of a structure of a computer device according to an embodiment of the present disclosure.

In the drawings, signs with the same last two digits correspond to the same elements. It should be noted that elements in the drawings are schematic and are not drawn to scale.

### Detailed Description

To make the objectives, technical solutions and advantages of the embodiments of the present disclosure clearer, technical solutions of the embodiments of the present disclosure will be clearly and completely described below in combination with the accompany drawings. Apparently, the described embodiments are a part of the embodiments of the present disclosure, not all of the embodiments. Based on the described embodiments of the present disclosure, all other embodiments obtained by those of skilled in the art without inventive efforts are covered by the scope of protection of the present disclosure.

Unless otherwise defined, all terms (including technical and scientific terms) used herein have the same meanings as commonly understood by those of skilled in the art to which the subject matter of the present disclosure belongs. It is further understood that terms as defined in commonly used dictionaries should be construed to have meanings consistent with their meanings in the context of the specification and related art and are not to be construed in an idealized or overly formal manner unless explicitly defined otherwise herein. As used herein, a statement that "connecting" or "coupling" two or more parts together should mean that the parts are joined together directly or by one or more intermediate components.

Reference herein to an "embodiment" means that a particular feature, structure, or characteristic described in combination with the embodiment may be included in at least one embodiment of the present application. The appearance of the phrase "embodiment" in various positions in the specification does not necessarily refer to the same embodiment, nor is it an independent or alternative embodiment that is mutually exclusive from other embodiments. It is explicitly and implicitly understood by those skilled in the art that the embodiments described herein may be combined with other embodiments.

Herein, the term "and/or" is only an association relationship describing association objects, and means that there may be three relationships, for example, A and/or B, which may mean that A exists, A and B simultaneously exist, and B exists. In addition, the symbol "/" herein generally indicates that objects before and after the symbol "/" have an "or" relationship.

Furthermore, in all embodiments of the present disclosure, terms such as "first" and "second" are used only to distinguish one component (or a portion of a component) from another component (or another portion of the component).

In the description of the present application, unless otherwise specified, the meaning of "a plurality" refers to two or more, and similarly, "a plurality of sets" refers to two or more sets.

In order to make those skilled in the art better understand the solutions of the present application, the technical solutions in the embodiments of the present application will be clearly and completely described below with reference to the accompanying drawings.

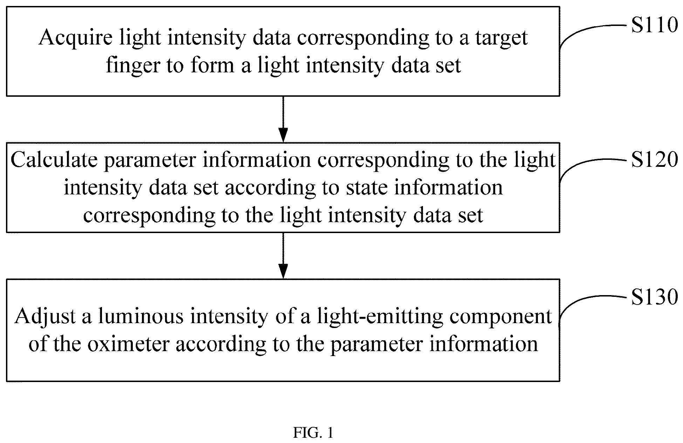

Based on the problems existing in the existing art, FIG. 1 illustrates schematically a flowchart of a method for adjusting an oximeter according to an embodiment of the present disclosure. As shown in FIG. 1, a specific process of the method for adjusting an oximeter includes the following acts S110 to S130.

In the act S110, light intensity data corresponding to a target finger is acquired to form a light intensity data set.

Specifically, the light intensity data set includes a plurality of light intensity data, and the light intensity data is light intensity data transmitted through the target finger and detected by a light receiving component of the oximeter.

**In** a specific implementation, first, the target finger is placed on the oximeter, the light intensity data transmitted through the target finger is detected by a photodiode of the oximeter.

An implementation of acquiring the light intensity data corresponding to the target finger to form the light intensity data set includes: acquiring light intensity data corresponding to the target finger and sequentially forming the light intensity data set of which the state information is the target preset threshold.

Specifically, the target preset threshold at least including a first target preset threshold and a second target preset threshold is set, and a first light intensity data set for the first target preset threshold and a second light intensity data set for the second target preset threshold are sequentially formed by continuously acquiring the light intensity data corresponding to the target finger.

Exemplarily, the first target preset threshold is 100 and the second target preset threshold is 30. By acquiring the light intensity data corresponding to the target finger and dividing the collected light intensity data of the target finger, a first light intensity data set of which state information is the first target preset threshold is sequentially formed, and a light intensity data set of which state information is the second target preset threshold is sequentially formed. For example, if there are 150 collected light intensity data corresponding to the target finger, the light intensity data 1-100 form a first light intensity data set, and the quantity of the remaining light intensity data 101-150 does not meet the first preset threshold and cannot form a first light intensity data set. Alternatively, the light intensity data 1-30 may form a second light intensity data set, the light intensity data 31-60 may form a second light intensity data set, the light intensity data 61-90 may form a second light intensity data set, the light intensity data 91-120 may form a second light intensity data set, and the light intensity data 121-150 may form a second light intensity data set.

In the act S120, parameter information corresponding to the light intensity data set is calculated according to state information corresponding to the light intensity data set.

The state information includes the quantity of the light intensity data included in the light intensity data set and/or a duration for which the light intensity data set is collected, wherein the parameter information includes a perfusion index and/or an average light intensity.

Perfusion Index (PI) indicates a pulsatile blood flow situation, that is, an ability for perfusing a blood flow. The larger the pulsatile blood flow is, the more pulsatile components and the larger the PI value. Therefore, both the measurement site (skin, nails, bones, etc.) and the patient's own blood perfusion (arterial blood flow situation) affect the PI value. Since the heart rate and the arterial blood pressure (the pulse arterial blood flow) are affected by sympathetic nerves, the PI value is indirectly affected by the body's neuromodulation system or mental state.

The average light intensity refers to an average value of the light intensity transmitted through the target finger and detected by the photodiode of the oximeter. The average light intensity indirectly reflects the blood flow state of the human body.

In an implementation, determining the parameter information corresponding to the light intensity data set according to state information corresponding to the light intensity data set includes: configuring a pre-associated adjustment parameter type according to the target preset threshold, and calculating parameter information corresponding to the adjustment parameter type according to the acquired light intensity data set.

Specifically, a perfusion index is determined as the adjustment parameter type according to the first target preset threshold, and a corresponding first perfusion index is calculated according to the first light intensity data set; and an average light intensity is determined as the adjustment parameter type according to the second target preset threshold, and a corresponding first average light intensity is calculated according to the second light intensity data set.

For example, when there are 100 light intensity data included in the formed first light intensity data set, the corresponding first perfusion index is calculated based on the first light intensity data set. When there are 30 light intensity data included in the formed second light intensity data set, the corresponding first average light intensity is calculated based on the second light intensity data set.

The first perfusion index is calculated in the following way: first an average DC corresponding to 100 light intensity data included in the first light intensity data set is calculated, then a difference AC corresponding to a maximum light intensity value and a minimum light intensity value corresponding to the 100 light intensity data included in the first light intensity data set is calculated, and the first perfusion index is determined by multiplying the average DC by the difference AC between the maximum light intensity value and the minimum light intensity value. The first average light intensity is calculated in the following way: an average DC corresponding to the 30 light intensity data included in the second light intensity data set is calculated.

In this embodiment, when the quantity of the acquired light intensity data does not meet the first target preset threshold but meets the second target preset threshold, the first average light intensity corresponding to the 30 light intensity data in the light intensity data set is acquired, the current gear is adjusted according to a user's finger condition, thereby adjusting the light intensity of a finger for transmitting light, such as a bright finger (thin finger is easy to transmit light) and dark finger (fat finger is difficult to transmit light), reducing a time length for adjusting to a proper light intensity and improving the measurement accuracy of the oximeter. As the quantity of the acquired light intensity data increases, when the quantity of the light intensity data meets the first predetermined target threshold, the first perfusion index corresponding to the light intensity data is calculated. The current gear of the oximeter is then adjusted based on the first perfusion index. Such adjustment eliminates the impact of the human body's intrinsic environment on the measurement accuracy of the oximeter, thereby improving the accuracy of the oximeter.

In another implementation, determining the parameter information corresponding to the light intensity data set according to state information corresponding to the light intensity data set includes: when the state information of the acquired light intensity data set meets N times the target preset threshold, configuring a pre-associated adjustment parameter type according to a target preset threshold, and calculating parameter information corresponding to the adjustment parameter type according to a latest acquired light intensity data subset, wherein the light intensity data set includes N light intensity data subsets, state information of each light intensity data subset is the target preset threshold, and N is an integer greater than or equal to 1.

Specifically, when t the state information of the acquired light intensity data set meets A times the first target preset threshold, the perfusion index is determined as the adjustment parameter type according to the first target preset threshold, and a corresponding second perfusion index is calculated according to a latest acquired light intensity data subset. When the state information of the acquired light intensity data set meets B times the second target preset threshold, the average light intensity is determined as the adjustment parameter type according to the second target preset threshold, and a corresponding second average light intensity is calculated according to the latest acquired light intensity data subset.

In this embodiment, the first target preset threshold is 100, the second target preset threshold is 30, and the light intensity data set is formed by acquiring light intensity data corresponding to the target finger. When the state information of the acquired light intensity data set meets A times the first target preset threshold, for example, there are 150 collected light intensity data corresponding to the target finger, a light intensity data set consisting of the light intensity data 1-100 meets 1 time the first target preset threshold. In this case, the perfusion index has been calculated according to the light intensity data subset consisting of the light intensity data 1-100. As the quantity of the acquired light intensity data continuously increases, for example, to 200 (i.e., the quantity of the light intensity data set meets 2 times the first target preset threshold), the corresponding second perfusion index is calculated according to a light intensity data subset consisting of the light intensity data 101-200. If there are 150 collected light intensity data corresponding to the target finger, the light intensity data 1-30 forms a second light intensity data set, the light intensity data 31-60 forms a second light intensity data set, the light intensity data 61-90 forms a second light intensity data set, the light intensity data 91-120 forms a second light intensity data set, and the light intensity data 121-150 forms a second light intensity data set. As the quantity of the light intensity data continuously increases, for example, to 180 (i.e., the state information of the light intensity data set meets 6 times the second target preset threshold), the second average light intensity corresponding to the light intensity data subset formed by the light intensity data 151-180 is calculated.

In the abovementioned embodiment, if the state parameter is a duration for collecting the light intensity data set, the first target preset threshold is a duration of one cycle of the pulse waves, and the second target preset threshold is less than the duration of one cycle of the pulse waves. If the state parameter is the quantity of collected light intensity data sets, the second target preset threshold is less than the first target preset threshold.

Since the oximeter requires a duration of at least one cycle of the pulse waves (normally 60/min) to calculate the PI value, and when 100 light intensity data are acquired, the duration corresponding to the 100 light intensity data is just the duration of one cycle of the pulse waves, the parameter information corresponding to the light intensity data set can be determined according to the quantity of collected light intensity data sets or the duration for collecting the light intensity data set.

In the act S130, a luminous intensity of a light-emitting component of the oximeter is adjusted according to the parameter information.

As a specific implementation, the current gear of the oximeter is adjusted according to a relationship between the parameter information and the preset parameter information.

The preset parameter information includes a preset perfusion index and a preset average light intensity.

In a specific implementation, adjusting the current gear of the oximeter according to the relationship between the parameter information and the preset parameter information includes: adjusting the current gear of the oximeter according to a relationship between the perfusion index and the preset perfusion index.

Specifically, the current gear of the oximeter is increased when the perfusion index is less than the first preset perfusion index; and the current gear of the oximeter is decreased when the perfusion index is greater than the second preset perfusion index.

The first preset perfusion index is 0.4, and the second preset perfusion index is 1, wherein 0.4 is a weak perfusion index, 1 is a strong perfusion index, and in the weak perfusion index and the strong perfusion index, the measurements of blood oxygen value are inaccurate.

The perfusion index is calculated, and then the current gear of the oximeter is adjusted according to the perfusion index. Because the perfusion index can stably reflect the blood flow state of the human body and is less affected by the external environment, for example, the PI value decreases with reduced peripheral blood flow in cold fingers, measurement inaccuracy caused by the body's physiological response to environmental temperature changes is effectively mitigated by adjusting the current gear of the oximeter according to the calculated PI value.

In another specific implementation, adjusting the current gear of the oximeter according to the relationship between the average light intensity and the preset average light intensity includes: adjusting the current gear of the oximeter according to the relationship between the perfusion index and the preset perfusion index.

Specifically, the current gear of the oximeter is increased when the average light intensity is less than the first preset average light intensity, and the current gear of the oximeter is decreased when the average light intensity is greater than the second preset average light intensity.

By calculating the average light intensity, the current gear of the oximeter is adjusted according to the average light intensity, so as to avoid the influence of bright fingers (thin fingers are easy to transmit light) and dark fingers (fat fingers are difficult to transmit light) on the received light intensity and on the measurement accuracy of the oximeter. Besides, the influence of ambient light on the measurement accuracy of the oximeter can be eliminated.

It should be noted that, in the abovementioned embodiments, in the process of increasing and decreasing the current gear of the oximeter, increasing or decreasing the current gear of the oximeter is performed each time by one current gear.

It should be noted that, in embodiments of the present disclosure, the current gear of the oximeter includes 5 gears, wherein a current value at the first gear is a current value required for a maximum light intensity that a photodiode in the oximeter is capable of receiving when there is no finger in the oximeter, the current value at the first gear is a minimum current value; a current value at the fifth gear is a current value required for a maximum light intensity that the photodiode is capable of receiving when a fattest (darkest) finger is put into the oximeter according to big data obtained by collecting finger thickness of a plurality of persons, the current value at the fifth gear is the maximum current value; current values at other gears are obtained by equally dividing a range between the maximum current value and the minimum current value.

The method for adjusting an oximeter according to an embodiment of the present disclosure includes: acquiring light intensity data corresponding to a target finger to form a light intensity data set, wherein the light intensity data is light intensity data transmitted through the target finger and detected by a light receiving component of the oximeter; calculating parameter information corresponding to the light intensity data set according to state information corresponding to the light intensity data set; and adjusting luminous intensity of a light-emitting component of the oximeter according to the parameter information, wherein the parameter information corresponding to the calculated light intensity data set includes an average light intensity and a perfusion index. Adjusting the current gear of the oximeter according to the average light intensity can eliminate the influences of bright fingers, dark fingers and ambient light on the measurement accuracy of the oximeter. The current gear of the oximeter is adjusted based on the perfusion index, which eliminates the impact of the human body's intrinsic environment on the measurement accuracy of the oximeters, thereby not only eliminating the influence of both finger thickness and skin color, but also eliminating the influences of environmental factors, on the measurement accuracy of the oximeter. In the present invention, different adjustment parameter types are determined according to the state information of the light intensity data set, which enhances the adjustment efficiency for the light intensity, so that the oximeter can stably output the value in a short time, and the oximeter can output the value faster.

In a specific implementation, before acquiring the light intensity data corresponding to the target finger, the method further includes: adjusting the current gear of the oximeter to an initial gear that is an intermediate current gear of the oximeter.

Before acquiring the light intensity data corresponding to the target finger, adjusting the current gear of the oximeter to an initial gear that is an intermediate current gear of the oximeter at first, which can ensure the accuracy of the light intensity data acquired later, reduce unnecessary adjustment of the current gear, and improve the efficiency of outputting a stable value by the oximeter.

Furthermore, the method for adjusting an oximeter disclosed in the present application further includes: acquiring a preset quantity of light intensity data sets in a sliding window way; calculating an average value of the light intensity data sets; and determining that the finger falls off and outputting alarm information when the average value is greater than a first preset value and the current gear is a lowest gear.

Specifically, the light intensity data corresponding to the target finger at the lowest gear is acquired in the preset sliding window way. When a sliding window meets a preset sliding window, an average value of a light intensity data set corresponding to each sliding window is sequentially determined. When the average value is greater than the first preset value and the current gear is the lowest gear, it is determined that the finger falls off, and the alarm information is output.

When the current gear is adjusted based on the light intensity data set, in a process of acquiring the light intensity data corresponding to a next target finger, it is required to determine state information of the target finger. That is, if the target finger falls off while the light intensity data set corresponding to the target finger is acquired, it is required to acquire the light intensity data corresponding to the target finger again. The way of determining whether the target finger falls off includes: acquiring light intensity data corresponding to the target finger at a lowest gear in the preset sliding window way; sequentially determining the average value of light intensity data set corresponding to each sliding window when the sliding window meets the preset sliding window, and determining state information of the target finger according to the average value of light intensity data corresponding to each sliding window.

The preset sliding window way is as follows: acquiring light intensity data corresponding to windows 1-8, 2-9, 3-10, ..., by sliding the windows, and the quantity of sliding windows is related to a target preset threshold and light intensity data included in the sliding windows. For example, if the target preset threshold equals to 30, there are 8 light intensity data included in the sliding window, in this case, there are 23 sliding windows. When an average value of light intensity data included in a sliding window fluctuates greatly, the light intensity data collected in the sliding window indicates that the target finger falls off.

In addition, in the embodiment of the present disclosure, by adjusting the current of the oximeter, it can be ensured that the oximeter outputs a stable waveform, thereby accurately detecting changes in human blood oxygen based on the waveform output by the oximeter.

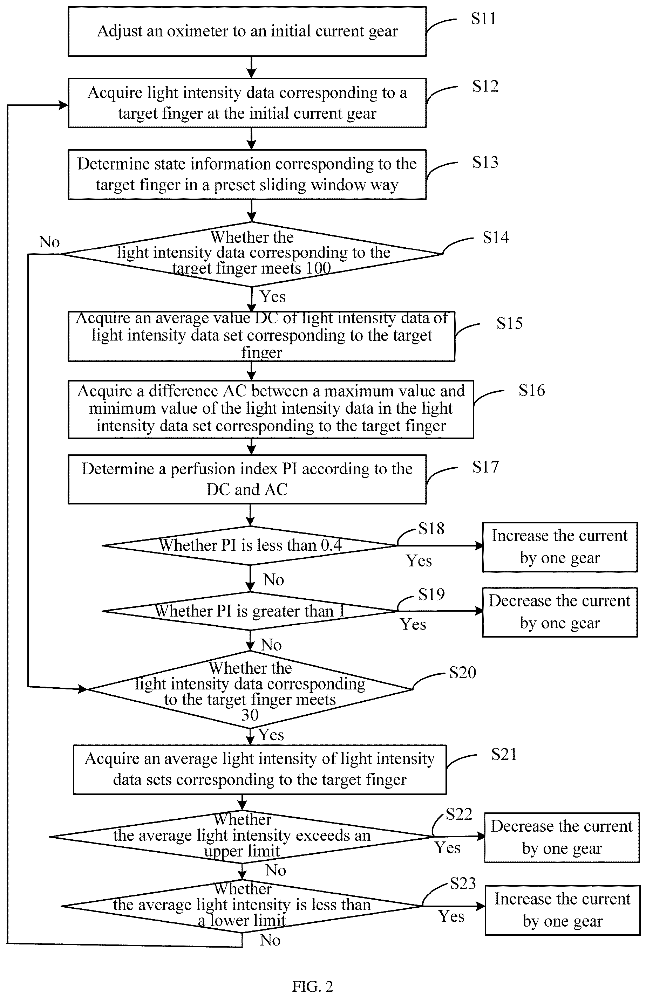

A specific example is illustrated in detail below. As shown in FIG. 2, firstly, an act S11 is performed, that is, an oximeter is adjusted to an initial current gear that is an intermediate current gear of the oximeter. Then an act S12 is performed, that is, light intensity data corresponding to a target finger at the initial current gear is acquired. In an act S13, state information of the target finger is determined according to a preset sliding window way. A specific process of the act S13 includes: acquiring the light intensity data corresponding to the target finger at the lowest gear in the preset sliding window way at first, then sequentially determining an average value of light intensity data set corresponding to each sliding window, and determining the state information of the target finger according to the average value of light intensity data corresponding to each sliding window. If determining the state information of the target finger is not falling off, an act S14 is performed. In the act S14, whether the acquired light intensity data corresponding to the target finger meets a target preset threshold is determined. If the acquired light intensity data corresponding to the target finger meets a first target preset threshold (100), an act S15 is performed. If the acquired light intensity data corresponding to the target finger does not meet the first target preset threshold, the process jumps to an act S20. In the act S20, whether the light intensity data corresponding to the target finger meets a second target preset threshold (30) is determined. In the act S15, an average value DC of the light intensity data sets corresponding to the target finger is acquired. In an act S16, a difference AC between a maximum value and a minimum value of light intensity data in the light intensity data set corresponding to the target finger is acquired. In an act S17, a perfusion index is determined according to the average value of the light intensity data sets and the maximum value and the minimum value of the light intensity data in the light intensity data set. In an act S18, whether the perfusion index is less than 0.4 is determined, the current gear is increased by one gear if determining that the perfusion index is less than 0.4, he process proceeds to an act S19 when the perfusion index is greater than 0.4. In the act S19, whether the perfusion index is greater than 1 is determined, the current gear is increased by one gear if determining that the perfusion index is greater than 1, the process proceeds to an act S20 if determining that the perfusion index is less than 1. In the act S20, whether light intensity data included in the light intensity data set meets a second target preset threshold is determined, the process proceeds to an act S21 if determining light intensity data included in the light intensity data set meets the second target preset threshold. In the act S21, an average light intensity of light intensity data corresponding to the second target preset threshold is calculated, and an act S22 is performed according to the calculated average light intensity of the light intensity data. In the act S22, whether the average light intensity exceeds an upper limit is determined. If the average light intensity exceeds the upper limit, the current is decreased by one gear. If the average light intensity does not exceed the upper limit, the process proceeds to an act S23. In the act S23, whether the average light intensity is less than a lower limit is determined; if the average light intensity is less than the lower limit, the current is increased by one gear; if the average light intensity is not less than the lower limit, the process returns to the act S12 to acquire a next light intensity data set and continue the cyclic adjustment as described above.

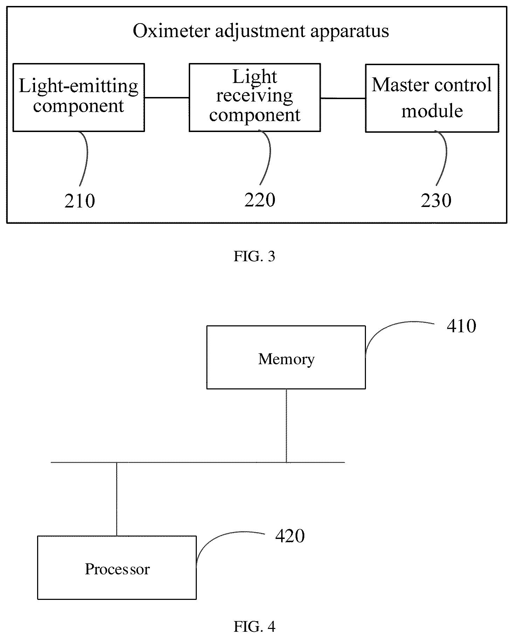

Based on the abovementioned embodiments, an oximeter adjustment apparatus is also provided in an embodiment of the present disclosure. As shown in FIG. 3, the oximeter adjustment apparatus includes: a light-emitting component 210, a light receiving component 220, and a master control module 230 connected to the light-emitting component and the light receiving component; the master control module 230 is configured to acquire light intensity data received by the light receiving module, form a light intensity data set, calculate parameter information corresponding to the light intensity data set according to state information corresponding to the light intensity data set, and adjust a luminous intensity of the light-emitting component according to the parameter information.

The oximeter adjustment apparatus according to the embodiment of the present disclosure includes the light-emitting component, the light receiving component, and the master control module connected to the light-emitting component and the light receiving component; The master control module is configured to acquire light intensity data received by the light receiving module, form a light intensity data set, calculate parameter information corresponding to the light intensity data set according to state information corresponding to the light intensity data set, and adjust a luminous intensity of the light-emitting component according to the parameter information. The parameter information corresponding to the calculated light intensity data set includes an average light intensity and a perfusion index. Adjusting the current gear of the oximeter according to the average light intensity can eliminate influences of bright fingers, dark fingers and ambient light on the measurement accuracy of the oximeter. The current gear of the oximeter is adjusted based on the perfusion index, which eliminates the impact of the human body's intrinsic environment on the measurement accuracy of the oximeter, thereby not only eliminating the influences of finger thickness and skin color, but also eliminating the influences of environmental factors, on the measurement accuracy of the oximeter. In the present invention, different adjustment parameter types are determined according to the state information of the light intensity data set, which enhances the adjustment efficiency for the light intensity, so that the oximeter can stably output the value in a short time, and the oximeter can output the value faster.

A computer device is also provided in an embodiment of the present application. Referring to FIG. 4 for details, FIG. 4 is a block diagram of a basic structure of a computer device according to the embodiment.

The computer device includes a memory 410 and a processor 420 communicatively connected to each other by a system bus. It should be noted that only a computer device having components 410 and 420 is shown in the figure, but it should be understood that it is not required that the computer device is implemented by all of the components shown, and it may be implemented by more or fewer components instead. Those skilled in the art can understand that the computer device herein is a device capable of automatically performing numerical calculation and/or information processing according to preset or stored instructions, and its hardware includes, but is not limited to, a microprocessor, an Application Specific Integrated Circuit (ASIC), a Field-Programmable Gate Array (FPGA), a Digital Signal Processor (DSP), an embedded device and the like.

The computer device may be a computing device such as a desktop computer, a notebook, a palm computer and a cloud server. The computer device may perform human-computer interaction with the user through a keyboard, a mouse, a remote control, a touch pad, or a voice-controlled device, etc.

The memory 410 at least includes one type of readable storage medium including a non-volatile memory or a volatile memory, such as a flash memory, a hard disk, a multimedia card, a card-type memory (e.g., an SD or DX memory, etc.), a Random Access Memory (RAM), a Read-Only Memory (ROM), an Erasable Programmable Read-Only Memory (EPROM), an Electrically Erasable Programmable Read-Only Memory (EEPROM), a Programmable Read-Only Memory (PROM), a magnetic memory, a magnetic disk, an optical disk and the like, wherein the RAM may include a static RAM or a dynamic RAM. In some embodiments, the memory 410 may be an internal storage unit of a computer device, e.g. a hard disk or memory of the computer device. In some other embodiments, the memory 410 may also be an external storage device of the computer device, such as a plug-in hard disk, a Smart Media Card (SMC), a Secure Digital (SD) card, or a Flash Card (Flash Card) provided on the computer device. Of course, the memory 410 may also include both an internal storage unit of the computer device and an external storage device thereof. In this embodiment, the memory 410 is generally configured to store an operating system and various types of application software installed in the computer device, such as program codes for the abovementioned method. Furthermore, the memory 410 may also be configured to temporarily store various types of data that have been output or will be output.

The processor 420 is generally configured to perform the overall operations of the computer device. In this embodiment, the memory 410 is configured to store program codes or instructions, and the program codes include computer operation instructions, and the processor 420 is configured to execute the program codes or instructions stored in the memory 410 or process data, such as program codes for executing the abovementioned method.

The bus may be an Industry Standard Architecture (ISA) bus, a Peripheral Component Interconnect (PCI) bus, an Extended Industry Standard Architecture (EISA) bus, or the like. The bus system can be classified into an address bus, a data bus, a control bus, etc. For convenience of illustration, only one bold line is used in the figure, but it does not indicate that there is only one bus or one type of bus.

A computer readable medium is also provided in another embodiment of the present application, which may be a computer readable signal medium or a computer readable medium. A processor in the computer reads computer readable program codes stored in a computer readable medium, so that the processor can perform functional actions specified in each act, or combinations of acts, in the abovementioned method. An apparatus that implements functional actions defined in each block or a combination of blocks of the block diagram is generated.

The computer readable medium includes, but is not limited to, an electronic, magnetic, optical, electromagnetic, infrared memory or semiconductor system, device, or apparatus, or any suitable combination of the foregoing. The memory is configured to store program codes or instructions, wherein the program codes include computer operation instructions, and the processor is configured to execute the program codes or instructions of the abovementioned method stored on the memory.

The definitions of the memory and the processor can be referred to in the description of the abovementioned embodiments of the computer device, which is not repeated herein.

In several embodiments according to the present application, it should be understood that the disclosed systems, apparatuses, and methods may be implemented in other ways. For example, the apparatus embodiment described above is only schematic. For example, the division of the modules or units is only logical function division, and there may be other division ways in practical implementation. For example, a plurality of units or assemblies may be combined or integrated into another system, or some features may be omitted or not executed. In addition, the displayed or discussed coupling or direct coupling or communication connection between each other may be indirect coupling or communication connection between apparatuses or units via some interfaces, and may be electrical, mechanical or in other forms.

Functional units or modules in embodiments of the present application may be integrated in one processing unit, the units may physically exist alone, or two or more units may be integrated in one unit. The abovementioned integrated unit may be implemented in the form of hardware, or software functional unit.

The integrated unit may be stored in a computer readable storage medium if implemented in the form of a software functional unit and sold or used as a stand-alone product. Based on such understanding, the technical solution of the present application, in essence, or the part contributing to the prior art, or the all or part of the technical solution, may be embodied in the form of a computer software product stored in a storage medium, including a number of instructions for enabling a computer device (which may be a personal computer, a server, or a network device) or a processor to perform all or part of the acts of the methods described in various implementations of the present application. The storage medium includes a USB disk, a removable hard disk, a Read-Only Memory (ROM), a Random Access Memory (RAM), a magnetic disk, or an optical disk that can store program codes.

Unless the context clearly dictates otherwise, the singular form of words used herein and in the appended claims includes the plural, and vice versa. Accordingly, references to the singular typically include the plural of the corresponding terms. Similarly, the terms "contain" and "include" are to be construed as inclusive rather than exclusive. Likewise, the terms "include" and "or" should be construed as inclusive unless such interpretation is expressly prohibited herein. Where the term "example" is used herein, particularly when it follows a set of terms, the "example" is merely exemplary and expository, and should not be considered exclusive or broad.

Further aspects and scope of adaptability will become apparent from the description provided herein. It should be understood that various aspects of the present application may be practiced alone or in combination with one or more other aspects. It should also be understood that the description and specific embodiments herein are intended for illustrative purposes only and are not intended to limit the scope of the present application.

Several embodiments of the present disclosure have been described in detail above, but it is apparent that various modifications and variations can be made to the embodiments of the present disclosure by those skilled in the art without departing from the spirit and scope of the present disclosure. The scope of the present disclosure is defined by the appended claims.

## Claims

1. A method for adjusting an oximeter, comprising:
acquiring light intensity data corresponding to a target finger to form a light intensity data set, wherein the light intensity data is light intensity data transmitted through the target finger and detected by a light receiving component of the oximeter;
calculating parameter information corresponding to the light intensity data set according to state information corresponding to the light intensity data set; and
adjusting a luminous intensity of a light-emitting component of the oximeter according to the parameter information.

2. The method of claim **1,** wherein the state information comprises a quantity of light intensity data comprised in the light intensity data set and/or a duration for which the light intensity data set is collected, wherein a type of the parameter information comprises a perfusion index and/or an average light intensity.

3. The method of claim 1, wherein acquiring the light intensity data corresponding to the target finger to form the light intensity data set comprises:
collecting the light intensity data corresponding to the target finger, and sequentially forming the light intensity data set of which the state information is a target preset threshold;
determining the parameter information corresponding to the light intensity data set according to the state information corresponding to the light intensity data set, comprises:
configuring a pre-associated adjustment parameter type according to the target preset threshold, and calculating parameter information corresponding to the adjustment parameter type according to the acquired light intensity data set.

4. The method of claim 3, wherein the target preset threshold comprises, at least, a first target preset threshold and a second target preset threshold;
sequentially forming the light intensity data set of which the state information is the target preset threshold comprises:
sequentially forming a first light intensity data set of which state information is the first target preset threshold, and sequentially forming a second light intensity data set of which state information is the second target preset threshold;
configuring the pre-associated adjustment parameter type according to the target preset threshold, and calculating the parameter information corresponding to the adjustment parameter type according to the acquired light intensity data set comprises:
determining a perfusion index as the adjustment parameter type according to the first target preset threshold, and calculating a corresponding first perfusion index according to the first light intensity data set; and
determining an average light intensity as the adjustment parameter type according to the second target preset threshold, and calculating a corresponding first average light intensity according to the second light intensity data set.

5. The method of claim 4, wherein calculating the corresponding first perfusion index according to the first light intensity data set comprises:
calculating an average value of the first light intensity data set;
calculating a difference between a maximum light intensity value and a minimum light intensity value corresponding to light intensity data comprised in the first light intensity data set; and
determining the first perfusion index by the average value and the difference between the maximum light intensity value and the minimum light intensity value.

6. The method of claim 1, wherein calculating the parameter information corresponding to the light intensity data set according to the state information corresponding to the light intensity data set comprises:
when the state information of the acquired light intensity data set meets N times a target preset threshold, configuring a pre-associated adjustment parameter type according to the target preset threshold, and calculating parameter information corresponding to the adjustment parameter type according to a latest acquired light intensity data subset, wherein the light intensity data set comprises N light intensity data subsets, state information of each light intensity data subset is the target preset threshold, and N is an integer greater than or equal to 1.

7. The method of claim 6, wherein the target preset threshold comprises, at least, a first target preset threshold and a second target preset threshold;
when the state information of the acquired light intensity data set meets N times the target preset threshold, configuring the pre-associated adjustment parameter type according to the target preset threshold, and calculating the parameter information corresponding to the adjustment parameter type according to the latest acquired light intensity data subset, comprises:
when the state information of the acquired light intensity data set meets A times the first target preset threshold, determining a perfusion index as the adjustment parameter type according to the first target preset threshold, and calculating a corresponding second perfusion index according to the latest acquired light intensity data subset;
when the state information of the acquired light intensity data set meets B times the second target preset threshold, determining an average light intensity as the adjustment parameter type according to the second target preset threshold, and calculating a corresponding second average light intensity according to the latest acquired light intensity data subset.

8. The method of claim 4 or 7, wherein the state parameter is a duration for which the light intensity data set is collected, the first target preset threshold is a duration of one cycle of pulse waves, and the second target preset threshold is less than the duration of one cycle of pulse waves;
and/or,
the state parameter is a quantity of collected light intensity data sets, and the second target preset threshold is less than the first target preset threshold.

9. The method of claim 1, wherein adjusting the luminous intensity of the light-emitting component of the oximeter according to the parameter information comprises:
adjusting a current gear of the oximeter according to a relationship between the parameter information and preset parameter information.

10. The method of claim 9, wherein the current gear of the oximeter comprises a plurality of gears, wherein a current value at a first gear is a current value required for a maximum light intensity that a photodiode in the oximeter is capable of receiving when there is no finger in the oximeter, the current value at the first gear is a minimum current value; a current value at a highest gear is a current value required for a maximum light intensity that the photodiode is capable of receiving when a preset dark finger is put into the oximeter, the current value at the highest gear is a maximum current value; current values at other gears are obtained by equally dividing a range between the maximum current value and the minimum current value.

11. The method of claim 9, wherein adjusting the current gear of the oximeter comprises increasing the current gear of the oximeter and decreasing the current gear of the oximeter, and increasing or decreasing the current gear of the oximeter is performed each time by one current gear.

12. The method of claim 4 or 7, wherein adjusting the luminous intensity of the light-emitting component of the oximeter according to the parameter information comprises:
adjusting a current gear of the oximeter according to a relationship between the perfusion index and a preset perfusion index; and/or,
adjusting the current gear of the oximeter according to a relationship between the average light intensity and a preset average light intensity.

13. The method of claim 12, wherein the preset perfusion index comprises a first preset perfusion index and a second preset perfusion index;
adjusting the current gear of the oximeter according to the relationship between the perfusion index and the preset perfusion index comprises:
increasing the current gear of the oximeter when the perfusion index is less than the first preset perfusion index; and
decreasing the current gear of the oximeter when the perfusion index is greater than the second preset perfusion index.

14. The method of claim 12, wherein the preset average light intensity comprises a first preset average light intensity and a second preset average light intensity;
adjusting the current gear of the oximeter according to the relationship between the average light intensity and the preset average light intensity comprises:
increasing the current gear of the oximeter when the average light intensity is less than the first preset average light intensity; and
decreasing the current gear of the oximeter when the average light intensity is greater than the second preset average light intensity.

15. The method of claim 9, further comprising:
acquiring a preset quantity of light intensity data sets in a sliding window way;
calculating average values of the light intensity data sets; and
determining that the finger falls off and outputting alarm information when an average value is greater than a first preset value and the current gear is a lowest gear.

16. An oximeter adjustment apparatus, comprising:
a light-emitting component, a light receiving component, and a master control module that is connected to the light-emitting component and the light receiving module;
the master control module is configured to acquire light intensity data received by the light receiving module to form a light intensity data set, calculate parameter information corresponding to the light intensity data set according to state information corresponding to the light intensity data set, and adjust a luminous intensity of the light-emitting component according to the parameter information.

17. An electronic apparatus, comprising a processor, and a memory having stored therein a computer program that, when executed by the processor, implements the method of any one of claims 1 to 15.

18. A computer readable storage medium having stored thereon a computer program that, when executed, implements the method of any one of claims 1 to 15.
